# EUROPEAN PATENT APPLICATION

(11) **EP 4 691 469 A1**
(43) Date of publication of application: **11.02.2026**
(21) Application number: 24784978.9
(22) Date of filing: 05.04.2024
(51) Int. Cl.: A61K 31/513, A61K 9/48, A61K 47/14, A61K 47/26, A61P 1/00, A61P 29/00

(54) **SELF-EMULSIFYING COMPOSITION AND SELF-EMULSIFYING PREPARATION CONTAINING SULFONAMIDE DERIVATIVE**

(30) Priority: 06.04.2023 JP 2023061839
(71) Applicant: EA Pharma Co., Ltd., Tokyo 1040042 (JP)
(72) Inventor: ONOSHITA Tomoya, Tokyo 104-0042 (JP); HIRANO Satoshi, Tokyo 104-0042 (JP); ARISAKA Harumi, Tokyo 104-0042 (JP); OKADA Nobuto, Tokyo 104-0042 (JP); ABURATANI Satoshi, Tokyo 104-0042 (JP); TSURUTA Atsushi, Tokyo 104-0042 (JP); MATSUDA Yukihiro, Tokyo 104-0042 (JP)
(74) Representative: Mewburn Ellis LLP
(86) International application number: PCT/JP2024/014042
(87) International publication number: WO 2024/210196

(57) **Abstract**

Provided is a pharmaceutical composition containing a sulfonamide derivative represented by formula (1), a pharmaceutically acceptable salt thereof, or an active metabolite thereof, and having an improved absorption during oral administration. Disclosed is a self-emulsifying composition comprising a sulfonamide derivative represented by formula (1), a pharmaceutically acceptable salt thereof, or an active metabolite thereof, and at least two solubilizing agents.

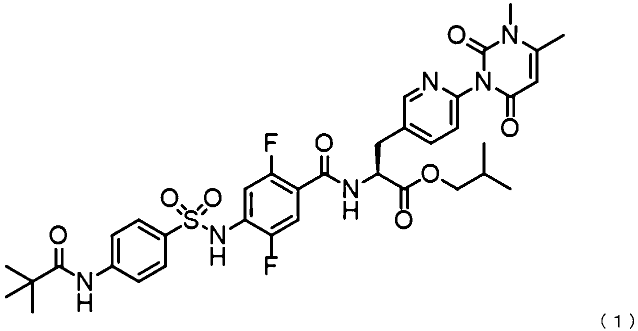

## Description

### Technical Field

The present disclosure relates to a self-emulsifying composition containing a sulfonamide derivative, and a self-emulsifying drug delivery system.

### Background Art

It is known that compounds having molecular weights of 500 or more generally have low water solubilities and have low membrane permeabilities at the time of oral administration (Donovan, M.D. et al. (1990) Absorption of polyethylene glycols 600 through 2000: The molecular weight dependence of gastrointestinal and nasal absorption. Pharm. Res. 7, 863-868). In the case of preparing a pharmaceutical product containing such a compound, there is a problem that absorbability decreases because of a low solubility or membrane permeability in the gastrointestinal tract after oral administration. For this reason, in order to ameliorate absorbability of a compound, various pharmaceutical measures have been taken. For example, a self-emulsifying drug delivery system composed of an oil, a surfactant, and the like (hereinafter referred to as "SEDDS") is also one of such measures. SEDDS is a formulation prepared by uniformly mixing, and dissolving or dispersing an active ingredient in the state of not containing water. In the case where SEDDS is orally administered, SEDDS is dispersed or dissolved in a water content in the gastrointestinal tract to form an emulsion, so that it can be expected that the solubility and the membrane permeability of the contained compound are improved and the absorbability is ameliorated.

It is disclosed that whether application of SEDDS is possible has been studied as studies to ameliorate membrane permeability or absorbability, for middle-molecular compounds having low membrane permeabilities in Japanese Patent Application Publication No. 2021-169476, for tetrahydro-pyrazolopyrimidinone compounds in Published Japanese Translation of PCT International Application No. 2022-540780, and for ω3 polyunsaturated fatty acids in Japanese Patent Application Publication No. 2021-107433.

It has been reported that a sulfonamide derivative represented by the following formula (1), a pharmaceutically acceptable salt thereof, or an active metabolite thereof has an α4 integrin inhibiting action and is useful as a pharmaceutical agent for use in treating or preventing inflammatory bowel diseases and the like (International Publication No. WO2017/135472). However, findings regarding membrane permeability or absorbability of this compound, which has a molecular weight of 500 or more, have not been satisfactory.

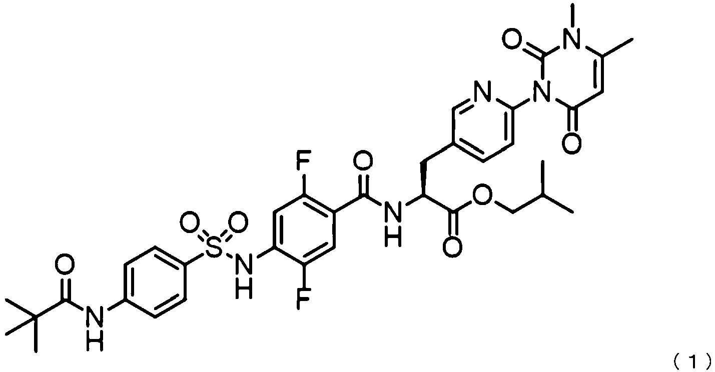

### Summary of Invention

### Problems to be solved by the invention

Even compounds having molecular weights of 500 or more have various physical properties such as solubility and membrane permeability, and it is not clear that using SEDDS can ameliorate the absorbability of a compound. In addition, it has not been clear what component may be combined with a compound to prepare SEDDS that can ameliorate solubility and membrane permeability.

Regarding the sulfonamide derivative represented by the above formula (1), a pharmaceutically acceptable salt thereof, or an active metabolite thereof, the studies of the present inventors revealed that there is room for ameliorating a blood exposure amount in the case of simply orally administering a powder or a suspension of this compound. That is, a further improvement in membrane permeability and absorbability has been desired.

The present disclosure has been found out by the present inventors earnestly studying in view of such situations, and one of objects of the present disclosure is to provide a pharmaceutical composition comprising a sulfonamide derivative represented by a formula (1), a pharmaceutically acceptable salt thereof, or an active metabolite thereof, and having an improved absorbability at the time of oral administration.

The present inventors have conducted various studies in order to solve the above problems, and found that absorbability of the sulfonamide derivative represented by the formula (1), a pharmaceutically acceptable salt thereof, or an active metabolite thereof can be ameliorated by preparing a self-emulsifying composition comprising: the sulfonamide derivative represented by the formula (1), a pharmaceutically acceptable salt thereof, or an active metabolite thereof; and at least two solubilizers, thus completing the invention.

Non-limiting aspects of the present disclosure provide the following technical ideas.
[1] A self-emulsifying composition comprising: a sulfonamide derivative represented by the following formula (1), a pharmaceutically acceptable salt thereof, or an active metabolite thereof; and at least two solubilizers 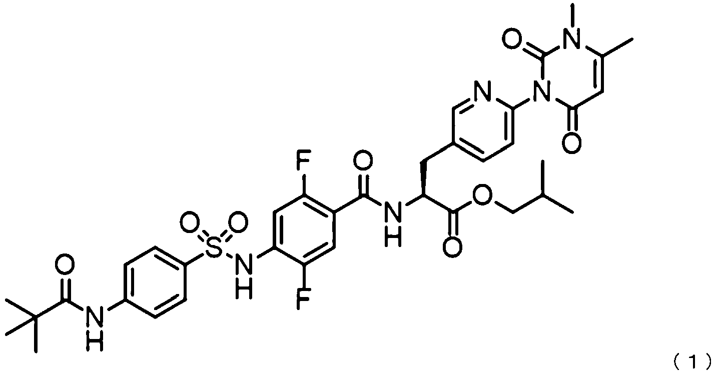
[2] The self-emulsifying composition according to the [1], wherein the at least two solubilizers comprise at least one surfactant and at least one auxiliary surfactant.
[3] The self-emulsifying composition according to the [1], wherein the solubilizers comprise at least two selected from a cyclodextrin or a derivative thereof, bile acid or a derivative thereof, a fatty alcohol, a fatty acid, or derivatives thereof, and a tocol derivative.
[4] The self-emulsifying composition according to the [2], wherein the surfactant comprises at least one selected from the group consisting of polyglyceryl-2 dioleate, polyglyceryl-3 dioleate, polyglyceryl-10 trioleate, propylene glycol monolaurate, propylene glycol monocaprylate, glyceryl monocaprylate, glyceryl monolaurate, glyceryl dilaurate, and glyceryl monolinoleate.
[5] The self-emulsifying composition according to the [2], wherein the auxiliary surfactant comprises at least one selected from the group consisting of polyoxyl 40 castor oil, polyoxyl 35 castor oil, PEG-25 trioleate, PEG-60 corn glycerides, PEG-60 almond oil, PEG-40 palm kernel oil, PEG-50 castor oil, PEG-50 hydrogenated castor oil, PEG-60 hydrogenated castor oil, PEG-8 caprylic/capric glycerides, lauroyl polyoxyl-32 glycerides, linoleoyl polyoxyl-6 glycerides, oleoyl polyoxyl-6 glycerides, stearoyl polyoxyl-32 glycerides, PEG-6 caprylic/capric glycerides, polyglyceryl-10 monooleate, polyglyceryl-10 laurate, polyglyceryl-15 hydroxystearate, α-tocopherol PEG-400 succinate, α-tocopherol PEG-1000 succinate, dl-α-tocopherol PEG-1000 succinate, and d-α-tocopherol PEG-1000 succinate.
[6] The self-emulsifying composition according to any one of the [2], [4], and [5], wherein
   a content of the surfactant is 0.1 times or more relative to % by mass of the sulfonamide derivative represented by the formula (1) or a pharmaceutically acceptable salt thereof, and
   a content of the auxiliary surfactant is 10 times or less relative to the sulfonamide derivative represented by the formula (1) or a pharmaceutically acceptable salt thereof.
[7] The self-emulsifying composition according to any one of the [2] and [4] to [6], wherein a ratio of the surfactant to the auxiliary surfactant is 1:1 to 8:1.
[8] The self-emulsifying composition according to any one of the [2] and [4] to [7], wherein the surfactant comprises propylene glycol monocaprylate.
[9] The self-emulsifying composition according to any one of the [2] and [4] to [8], wherein the auxiliary surfactant comprises polyoxyl 40 castor oil, polyoxyl 35 castor oil, or polyoxyethylene castor oil.
[10] The self-emulsifying composition according to any one of the [1] to [9], further comprising a cosolvent.
[11] A self-emulsifying drug delivery system in a form of a formulation suitable for oral administration, comprising the self-emulsifying composition according to any one of the [1] to [10].
[12] The self-emulsifying drug delivery system according to the [11], wherein the formulation is a capsule.

Moreover, as different one aspects of the present disclosure, the following technical ideas are also provided.
[2-1] A treatment agent or prevention agent for a disease, comprising a self-emulsifying composition comprising: a sulfonamide derivative represented by a formula (1), a pharmaceutically acceptable salt thereof, or an active metabolite thereof; and at least two solubilizers.
[2-2] The treatment agent or prevention agent according to the [2-1], wherein the disease is an inflammatory disease for which an α4 integrin-dependent adhesion process is involved in a pathology.
[2-3] The treatment agent or prevention agent according to the [2-1] or [2-2], wherein the disease is rheumatoid arthritis, ulcerative colitis, Crohn's disease, systemic lupus erythematosus, multiple sclerosis, Sjogren's syndrome, asthma, psoriasis, allergy, diabetes, cardiovascular disease, arteriosclerosis, restenosis, tumor growth, tumor metastasis, transplantation rejection, and/or human immunodeficiency virus infection.
[2-4] The treatment agent or prevention agent according to the [2-3], wherein the disease is ulcerative colitis or Crohn's disease.
[2-5] A method for using a self-emulsifying composition comprising: a compound represented by a formula (1), a pharmaceutically acceptable salt thereof, or an active metabolite thereof; and at least two solubilizers, for treating or preventing a disease.
[2-6] The method according to [2-5], wherein the disease is an inflammatory disease for which an α4 integrin-dependent adhesion process is involved in a pathology.
[2-7] The method according to the [2-5] or [2-6], wherein the disease is rheumatoid arthritis, ulcerative colitis, Crohn's disease, systemic lupus erythematosus, multiple sclerosis, Sjogren's syndrome, asthma, psoriasis, allergy, diabetes, cardiovascular disease, arteriosclerosis, restenosis, tumor growth, tumor metastasis, transplantation rejection, and/or human immunodeficiency virus infection.
[2-8] The method according to the [2-7], wherein the disease is ulcerative colitis or Crohn's disease.
[2-9] Use of the self-emulsifying composition according to any one of the [1] to [10], for producing a treatment agent or prevention agent of an inflammatory disease for which an α4 integrin-dependent adhesion process is involved in a pathology.
[2-10] The use according to the [2-9], wherein the disease is rheumatoid arthritis, ulcerative colitis, Crohn's disease, systemic lupus erythematosus, multiple sclerosis, Sjogren's syndrome, asthma, psoriasis, allergy, diabetes, cardiovascular disease, arteriosclerosis, restenosis, tumor growth, tumor metastasis, transplantation rejection, and/or human immunodeficiency virus infection.
[2-11] The use according to the [2-10], wherein the disease is ulcerative colitis or Crohn's disease.
[2-12] The self-emulsifying composition according to the [3], wherein the fatty acid or a derivative thereof is an amide ester of a fatty acid, an ester of a fatty acid and a hydroxy acid, a sugar ester or a sugar ester derivative of a fatty acid, an ester of a fatty acid and a propylene glycol, an ester of a fatty acid and a glycerol, an ester of a fatty acid and a polyglycerol, an ester of a fatty acid and an ethylene glycol, an ester of a fatty acid and a polyethylene glycol, or an ester of a fatty acid and a macrogol glycerin.

Furthermore, as different one aspects of the present disclosure, the following technical ideas are also provided.
[3-1] A self-emulsifying composition comprising: a sulfonamide derivative represented by a formula (1), a pharmaceutically acceptable salt thereof, or an active metabolite thereof; and at least two solubilizers, wherein a total content of the solubilizers is 10% by mass or more and 90% by mass or less relative to a total mass of the self-emulsifying composition.
[3-2] The self-emulsifying composition according to the [3-1], wherein the total content of the solubilizers is 20% by mass or more and 90% by mass or less relative to the total mass of the self-emulsifying composition.
[3-3] The self-emulsifying composition according to the [3-1], wherein the total content of the solubilizers is 30% by mass or more and 90% by mass or less relative to the total mass of the self-emulsifying composition.
[3-4] The self-emulsifying composition according to the [3-1], wherein the total content of the solubilizers is 40% by mass or more and 90% by mass or less relative to the total mass of the self-emulsifying composition.
[3-5] The self-emulsifying composition according to any one of the [3-1] to [3-4], wherein the solubilizers comprise at least one surfactant, and a content of the surfactant is 5% by mass or more and 85% by mass or less relative to the total mass of the self-emulsifying composition.
[3-6] The self-emulsifying composition according to any one of the [3-1] to [3-4], wherein the solubilizers comprise at least one surfactant, and a content of the surfactant is 10% by mass or more and 80% by mass or less relative to the total mass of the self-emulsifying composition.
[3-7] The self-emulsifying composition according to any one of the [3-1] to [3-5], wherein the solubilizers comprise at least one auxiliary surfactant, and a content of the auxiliary surfactant is 5% by mass or more and 85% by mass or less relative to the total mass of the self-emulsifying composition.
[3-8] The self-emulsifying composition according to any one of the [3-1] to [3-4] and [3-6], wherein the solubilizers comprise at least one auxiliary surfactant, and a content of the auxiliary surfactant is 10% by mass or more and 80% by mass or less relative to the total mass of the self-emulsifying composition.
[3-9] The self-emulsifying composition according to the [10], wherein the cosolvent is one selected from the group consisting of acetic acid, sorbitol, mannitol, glycerol, triacetin, triethyl citrate, N-methylpyrrolidone, N-hydroxyethyl pyrrolidone, polyvinylpyrrolidone, propylene carbonate, ethanol, and propylene glycol.
[3-10] The self-emulsifying composition according to the [3-9], wherein the cosolvent is ethanol or propylene glycol.
[3-11] The self-emulsifying composition according to any one of the [10], [3-9] and [3-10], wherein a content of the cosolvent is 10% by mass or less relative to the total mass of the self-emulsifying composition.
[3-12] The self-emulsifying composition according to the [3-11], wherein the content of the cosolvent is 8% by mass or less relative to the total mass of the self-emulsifying composition.
[3-13] The self-emulsifying composition according to the [3-12], wherein the content of the cosolvent is 5% by mass or less relative to the total mass of the self-emulsifying composition.
[3-14] The self-emulsifying composition according to any one of the [1] to [10], further comprising an additive.
[3-15] The self-emulsifying composition according to the [3-14], wherein the additive is at least one selected from the group consisting of an excipient, a lubricant, a binder, a pH adjuster, a flavoring agent, a thickener, a buffering agent, a colorant, a diluent, a preservative, and a sweetener.
[3-16] The self-emulsifying composition according to the [2] or [4], a mass ratio of the sulfonamide derivative represented by the formula (1) or a pharmaceutically acceptable salt thereof to the surfactant is 1:1 to 1:8.
[3-17] The self-emulsifying composition according to the [2] or [5], a mass ratio of the sulfonamide derivative represented by the formula (1) or a pharmaceutically acceptable salt thereof to the auxiliary surfactant is 1:1 to 1:8.
[3-18] A self-emulsifying composition comprising 10 to 1000 mg of a sulfonamide derivative represented by a formula (1), a pharmaceutically acceptable salt thereof, or an active metabolite thereof.

By providing a self-emulsifying composition comprising at least two solubilizers in addition to the sulfonamide derivative represented by the formula (1), a pharmaceutically acceptable salt thereof, or an active metabolite thereof, the present disclosure is capable of ameliorating absorbability of this compound, a pharmaceutically acceptable salt thereof, or an active metabolite thereof.

### Brief Description of Drawings

Fig. 1 is a graph showing a correlation between logarithmic values (Tube method Log(AUC_{0-2.25})) of AUC of a Tube method and logarithmic values (rat PO/PK Log(AUC)) of AUC of blood plasma concentrations.
Fig. 2 is a graph showing a correlation between logarithmic values (Tube method Log(AUC_{0-2.25})) of AUC of the Tube method and dissolution rates (%) at pH 2.4.
Fig. 3 is a graph showing a correlation between logarithmic values (Tube method Log(AUC_{0-2.25})) of AUC of the Tube method and dissolution rates (%) at pH 6.4.

### Description of Embodiments

Hereinafter, the present disclosure will be described in detail. Note that the embodiments described below are one aspects of the present disclosure and are not intended to limit the scope of the present disclosure.

In the present disclosure, a sulfonamide derivative (sometimes referred to as a "compound (1)" in the present Specification) represented by the following formula (1) is a compound that has an α4 integrin inhibiting action and is also referred to as 2-methylpropyl (2S)-2-{2,5-difluoro-4-[4-(2,2-dimethylpropylamide)phenylsulfonamide]benzamide}-3-{2-[1,6-dimethyl-2,4-dioxo-1,4-dihydropyrimidine-3(2H)-yl]pyridine-5-yl}propanoic acid. A person skilled in the art can recognize the scope of the present disclosure irrespective of the nomenclature as long as the above compound is indicated. This compound can be obtained by a publicly-known method, which is not limited to this but includes a method described in International Publication No. WO2017/135472 or the like, for example, as a synthesis method.

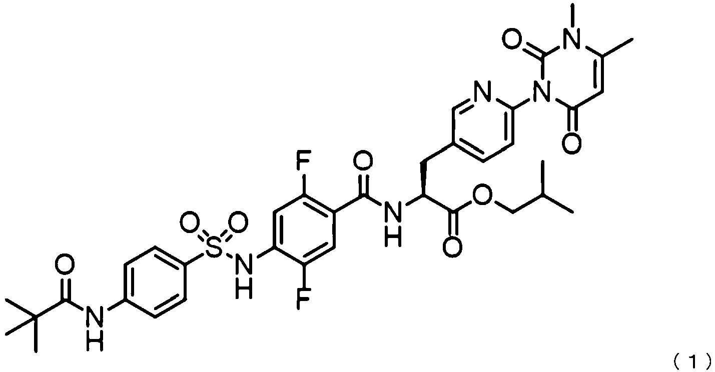

In the present disclosure, the "pharmaceutically acceptable salt" only has to be a pharmaceutically acceptable salt in which the sulfonamide derivative represented by the above formula (1) can be in the form of a salt while maintaining a pharmacological activity, and includes, for example, an ammonium salt; an alkali metal salt of sodium, potassium, or the like; an alkaline earth metal salt of calcium, magnesium, or the like; an aluminum salt; a zinc salt; a salt with an organic amine such as triethylamine, ethanolamine, morpholine, piperidine, or dicyclohexylamine; a salt with a basic amino acid such as arginine or lysine, for an acidic group such as a carboxyl group in the formula. In addition, for a basic group in the formula, the pharmaceutically acceptable salt includes a salt with an inorganic acid such as hydrochloric acid, sulfuric acid, or phosphoric acid; a salt with an organic carboxylic acid such as acetic acid, citric acid, benzoic acid, maleic acid, fumaric acid, tartaric acid, or succinic acid; and a salt with an organic sulfonic acid such as methanesulfonic acid or p-toluenesulfonic acid. In addition, as the method for forming a salt, a publicly-known method can be used as appropriate, and the method includes, for example, mixing the sulfonamide derivative represented by the formula (1) with a necessary acid or base in an appropriate ratio in a solvent or a dispersion medium, or the salt may be converted from another form of a salt by a method such as ion exchange.

In the present disclosure, the "active metabolite" refers to a compound group having an α4 integrin inhibiting action among compounds to which the sulfonamide derivative represented by the above formula (1) is converted by an enzyme, a gastrointestinal tract fluid, or the like under physiological conditions in vivo. The conversion reaction includes reductions such as oxidation, reduction, and hydrolysis. A non-limiting example of the active metabolite includes a compound represented by the following formula (2) (sometimes referred to as a "compound (2)" in the present Specification).

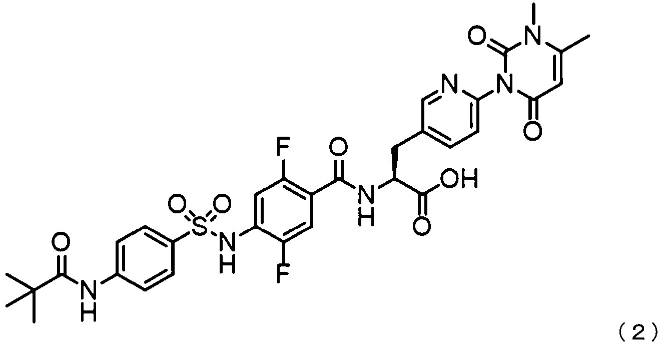

The "self-emulsifying composition" refers to a composition having a property of being capable of emulsifying to form an emulsion in an aqueous medium (self-emulsifying property) and includes a self-nanoemulsifying composition and a self-microemulsifying composition. An emulsion to be formed may have any size, and thus all of self- emulsifying drug delivery systems (SEDDS), self-microemulsifying drug delivery systems (SMEDDS), and self-nanoemulsifying drug delivery systems (SNEDDS) which are generally distinguished in accordance with the diameter of the emulsion belong to the scope of the self-emulsifying composition in the present disclosure.

The self-emulsifying composition in the present disclosure can be prepared by a publicly-known method. In addition, the properties of the self-emulsifying composition thus prepared can be evaluated by publicly-known methods as appropriate. In one non-limiting aspect of evaluating the properties of a self-emulsifying composition, a particle size and the like are known as indexes representing the physical properties of an emulsion, for example. As the method for evaluating a particle size, a conventionally known method can be used. For example, dynamic light scattering (DLS) can be used. In addition, as a parameter reflecting the particle size, a method for evaluating a turbidity by using a turbidimeter can also be used.

In the self-emulsifying composition of the present disclosure, the content of the sulfonamide derivative represented by the formula (1), a pharmaceutically acceptable salt thereof, or an active metabolite thereof is not particularly limited, but is preferably 10 mg or more, more preferably 50 mg or more, and even more preferably 100 mg or more, and preferably 1000 mg or less, more preferably 800 mg or more, and even more preferably 400 mg or less. In addition, in the self-emulsifying composition of the present disclosure, the sulfonamide derivative represented by the formula (1), a pharmaceutically acceptable salt thereof, or an active metabolite thereof is preferably contained in an amount of 10 to 1000 mg, more preferably contained in an amount of 50 to 800 mg, and even more preferably contained in an amount of 100 to 400 mg. When the total mass of the self-emulsifying composition of the present disclosure is considered as 100%, the content of the sulfonamide derivative represented by the formula (1), a pharmaceutically acceptable salt thereof, or an active metabolite thereof is preferably 1% by mass or more and more preferably 5% by mass or more, and preferably 50% by mass or less and more preferably 30% by mass or less.

The self-emulsifying composition of the present disclosure contains at least two solubilizers. A "solubilizer" means an appropriate medium that can enhance the solubility of a target solute. The solubilizers are not particularly limited within the scope of the present disclosure as long as they are publicly-known as solubilizers, but preferably include solubilizers belonging to any of a cyclodextrin (for example, β-cyclodextrin or γ-cyclodextrin), a cyclodextrin derivative (for example, sulfobutyl or hydroxypropyl ether), bile acid, a bile acid derivative (for example, cholate, ursodeoxycholate, chenodeoxycholate, taurochenodeoxycholate, taurourusodeoxycholate, glycochenodeoxycholate, or glycourusodeoxycholate), a fatty acid (for example, capric acid, caprylic acid, lauric acid, sarcosine acid, palmitic acid, myristic acid, stearic acid, oleic acid, linoleic acid, linolenic acid, ricinoleic acid, arachidonic acid, behenic acid, and pharmaceutically acceptable salts of these), a fatty acid derivative, an alcohol, a fatty alcohol (for example, stearyl alcohol), a fatty alcohol derivative (for example, polyethylene glycol octadecyl ether), as well as a tocol derivative. A preferable fatty acid derivative includes dioctyl sulfosuccinate sodium salt, sodium lauryl sulfate, an amide ester of a fatty acid (for example, lauric acid diethanolamide, sodium lauroyl sarcosinate, lauroylcarnitine, palmitoylcamitine, or myristoylcarnitine), an ester of a fatty acid or a fatty alcohol and a hydroxy acid (for example, sodium stearoyl lactylate or lauryl lactate), a sugar ester and a sugar ester derivative of a fatty acid (for example, glucose monocaprylate, diglucose monocaprylate, sucrose laurate, sorbitan monolaurate, sorbitan monopalmitate, sorbitan monooleate, sorbitan monostearate, sorbitan tristearate, sucrose myristate, or polyoxyethylene (20) sorbitan oleate (Polysorbate 80)), a fatty acid ester of a lower alcohol, an ester of a fatty acid and a propylene glycol, an ester of a fatty acid and a glycerol, an ester of a fatty acid and a polyglycerol, an ester of a fatty acid and an ethylene glycol, an ester of a fatty acid and a polyethylene glycol, an ester of a fatty acid and a macrogol glycerin (also referred to as a polyoxyglycerol), a triglyceride (for example, corn oil, almond oil, coconut oil, castor oil, hydrogenated castor oil, or hydrogenated coconut oil), a mixture of an ester of a fatty acid and a propylene glycol and an ester of a fatty acid and a macrogol glycerin, and a polyglycerized fatty acid.

As the total content of the solubilizers, the solubilizers may be blended in a desired ratio as long as an emulsion is formed and a self-emulsifying property is exhibited. For example, when the total mass of the self-emulsifying composition is considered as 100%, the total content of the solubilizers may be 10% by mass or more, 20% by mass or more, 30% by mass or more, 40% by mass or more, or 50% by mass or more. For example, when the total mass of the self-emulsifying composition is considered as 100%, the total content of the solubilizers may be 90% by mass or less. Although not limited to this, when the total mass of the self-emulsifying composition is considered as 100%, the total content of the solubilizers may be 10% by mass or more and 90% by mass or less. The total content of the solubilizers is preferably 20% by mass or more and 90% by mass or less, more preferably 30% by mass or more and 90% by mass or less, further preferably 40% by mass or more and 90% by mass or less, even more preferably 50% by mass or more and 90% by mass or less, and particularly preferably 60% by mass or more and 90% by mass or less.

In addition, in one aspect of the present disclosure, the solubilizers contain at least one surfactant and at least one auxiliary surfactant.

The "surfactant" refers to a group that exhibits lipophilicity among those belonging to the above solubilizers. Although not limited to this, as the index for lipophilicity, hydrophile-lipophile balance (HLB) can be used. HLB is obtained by equally dividing substances having no hydrophilic group as HLB=0 and substances having no lipophilic group as HLB=20, and can be calculated by a method known to a person skilled in the art such as the Griffin method, for example. In the present disclosure, HLB means a value calculated by the Griffin method. In general, the smaller the HLB is, the higher the lipophilicity is. Hence, in the self-emulsifying composition of the present disclosure, a solubilizer having a relatively small HLB can be selected as the surfactant as appropriate. For example, in the present disclosure, a solubilizer having HLB of 7 or less may be selected as the "surfactant". Specifically, the surfactant is an ester of a fatty acid and a propylene glycol, an ester of a fatty acid and a glycerol, or an ester of a fatty acid and a polyglycerol (HLB≤7), and is more specifically, polyglyceryl-2 dioleate, polyglyceryl-3 dioleate, polyglyceryl-10 trioleate, propylene glycol monolaurate, propylene glycol monocaprylate, glyceryl monocaprylate, glyceryl monolaurate, glyceryl dilaurate, glyceryl monolinoleate, or the like. These surfactants may be synthesized by a publicly-known method as appropriate, or may be commercially obtained. Non-limiting examples of the commercial products include commercially available products which have names such as Caprol, Capryol, Capmul, Lauroglycol, and Plurol Oleique in parts of their product names. One of these surfactants may be used alone, or two or more of these may be combined.

When the total mass of the self-emulsifying composition of the present disclosure is considered as 100%, the content of the surfactant is preferably 5% by mass or more and more preferably 10% by mass or more, and preferably 85% by mass or less and more preferably 80% by mass or less. In addition, when the content of the sulfonamide derivative represented by the formula (1), a pharmaceutically acceptable salt thereof, or an active metabolite thereof is considered as 1 in terms of the sulfonamide derivative represented by the formula (1), the content of the surfactant is 0.1 or more, preferably 0.5 or more, and more preferably 1.0 or more, and is 10 or less, preferably 9.5 or less, and more preferably 9 or less. In addition, the surfactant may be added such that a mass ratio between the sulfonamide derivative represented by the formula (1), a pharmaceutically acceptable salt thereof, or an active metabolite thereof and the surfactant becomes within a range of 1:1 to 1:10, and preferably 1:1 to 1:8.

The "auxiliary surfactant" refers to a group that exhibits hydrophilicity among those belonging to the above solubilizers. In general, the larger the above-mentioned HLB is, the higher the hydrophilicity is. Hence, in the self-emulsifying composition of the present disclosure, a solubilizer having a relatively large HLB may be selected as the auxiliary surfactant as appropriate. For example, in the present disclosure, a solubilizer having HLB of more than 7 may be selected as the "auxiliary surfactant". Specifically, the auxiliary surfactant is an ester of a fatty acid and a polyglycerol (HLB>7), an ester of a fatty acid and an ethylene glycol, an ester of a fatty acid and a polyethylene glycol, an ester of a fatty acid and a macrogol glycerin, a fatty alcohol derivative, or a tocol derivative, and is more specifically, polyoxyl 40 castor oil, polyoxyl 35 castor oil, PEG-25 trioleate, PEG-60 corn glycerides, PEG-60 almond oil, PEG-40 palm kernel oil, PEG-50 castor oil, PEG-50 hydrogenated castor oil, PEG-60 hydrogenated castor oil, PEG-8 caprylic/capric glycerides, lauroyl polyoxyl-32 glycerides, linoleoyl polyoxyl-6 glycerides, oleoyl polyoxyl-6 glycerides, stearoyl polyoxyl-32 glycerides, PEG-6 caprylic/capric glycerides, polyglyceryl-10 monooleate, polyglyceryl-10 laurate, polyglyceryl-15 hydroxystearate, or α-tocopherol succinate PEG (for example, α-tocopherol PEG-400 succinate, α-tocopherol PEG-1000 succinate, dl-α-tocopherol PEG-1000 succinate, or d-α-tocopherol PEG-1000 succinate). In addition, these auxiliary surfactants may be synthesized by a publicly-known method as appropriate, or may be commercially obtained. Non-limiting examples of the commercial products include commercially available products which have names such as Kolliphor, Cremophor, Labrafil, Labrasol, Gellucire, and TPGS in parts of their product names. One of these auxiliary surfactants may be used alone, or two or more of these may be combined.

Among the auxiliary surfactants listed as described above, the ester of a fatty acid and a macrogol glycerin includes polyoxyl 40 castor oil, polyoxyl 35 castor oil, PEG-25 trioleate, PEG-60 corn glycerides, PEG-60 almond oil, PEG-8 caprylic/capric glycerides, lauroyl polyoxyl-32 glycerides, linoleoyl polyoxyl-6 glycerides, oleoyl polyoxyl-6 glycerides, stearoyl polyoxyl-32 glycerides, and PEG-6 caprylic/capric glycerides, the ester of a fatty acid and a polyglycerol includes polyglyceryl-10 monooleate, polyglyceryl-10 laurate, and polyglyceryl-15 hydroxystearate, the ester of a fatty acid and a polyethylene glycol includes PEG-50 hydrogenated castor oil and PEG-60 hydrogenated castor oil, and the tocol derivative includes α-tocopherol PEG-400 succinate, α-tocopherol PEG-1000 succinate, dl-α-tocopherol PEG-1000 succinate, and d-α-tocopherol PEG-1000 succinate. As the ester of a fatty acid and a macrogol glycerin, the average number of moles added of a polyethylene glycol is preferably 20 or more and more preferably 32 or more. The ester of a fatty acid and a macrogol glycerin having an average number of moles added of 32 or more of a polyethylene glycol includes polyoxyl 40 castor oil, polyoxyl 35 castor oil, PEG-60 corn glycerides, PEG-60 almond oil, lauroyl polyoxyl-32 glycerides, and stearoyl polyoxyl-32 glycerides.

When the total mass of the self-emulsifying composition of the present disclosure is considered as 100%, the content of the auxiliary surfactant is preferably 5% by mass or more and more preferably 10% by mass or more, and preferably 85% by mass or less and more preferably 80% by mass or less. In addition, when the content of the sulfonamide derivative represented by the formula (1), a pharmaceutically acceptable salt thereof, or an active metabolite thereof is considered as 1 in terms of the sulfonamide derivative represented by the formula (1), the content of the auxiliary surfactant is 0.1 or more, preferably 0.5 or more, and more preferably 1.0 or more, and is 10 or less, preferably 9.5 or less, and more preferably 9 or less. In addition, the auxiliary surfactant may be added such that a mass ratio between the sulfonamide derivative represented by the formula (1), a pharmaceutically acceptable salt thereof, or an active metabolite thereof and the auxiliary surfactant becomes within a range of 1:1 to 1:10, and preferably 1:1 to 1:8.

The surfactant and the auxiliary surfactant may be blended in a desired ratio as long as an emulsion is formed and a self-emulsifying property is exhibited. For example, the ratio between the surfactant and the auxiliary surfactant may be 1:1 or more, 1:2 or more, or 1:3 or more. For example, the ratio between the surfactant and the auxiliary surfactant may be 1:10 or less, 1:9 or less, or 1:8 or less. Although not limited to this, the ratio between the surfactant and the auxiliary surfactant is, for example 1:1 or more and 1:10 or less, preferably 1:2 or more and 1:9 or less, and more preferably 1:3 or more and 1:8 or less.

In addition, the total content of the surfactant and the auxiliary surfactant may be blended in a desired ratio as long as an emulsion is formed and a self-emulsifying property is exhibited. For example, when the total mass of the self-emulsifying composition is considered as 100%, the total content of the surfactant and the auxiliary surfactant may be 10% by mass or more, 20% by mass or more, 30% by mass or more, 40% by mass or more, or 50% by mass or more. For example, when the total mass of the self-emulsifying composition is considered as 100%, the total content of the surfactant and the auxiliary surfactant may be 90% by mass or less. Although not limited to this, when the total mass of the self-emulsifying composition is considered as 100%, the total content of the surfactant and the auxiliary surfactant may be 10% by mass or more and 90% by mass or less. The total content of the surfactant and the auxiliary surfactant may be preferably 20% by mass or more and 90% by mass or less, more preferably 30% by mass or more and 90% by mass or less, further preferably 40% by mass or more and 90% by mass or less, even more preferably 50% by mass or more and 90% by mass or less, and particularly preferably 60% by mass or more and 90% by mass or less.

The self-emulsifying composition of the present disclosure may further comprise a cosolvent. The cosolvent includes acetic acid, sorbitol, mannitol, glycerol, triacetin, triethyl citrate, N-methylpyrrolidone, N-hydroxyethyl pyrrolidone, polyvinylpyrrolidone, propylene carbonate, ethanol, propylene glycol, and the like. These cosolvents may be synthesized by a publicly-known method as appropriate, or may be commercially obtained. One of these cosolvents may be used alone, or two or more of these may be combined.

In the self-emulsifying composition of the present disclosure, the cosolvent may be blended in a desired ratio as long as the self-emulsifying composition forms an emulsion and exhibits a self-emulsifying property. The ratio of the cosolvent is desirably as small as possible from the viewpoint of the commercial production of a self-emulsifying drug delivery system comprising the self-emulsifying composition of the present disclosure and use thereof in clinical sites. For example, although not limited to this, when the total mass of the self-emulsifying composition is considered as 100%, the content of the cosolvent may be 1% by mass or more and 15% by mass or less. The cosolvent may be preferably 1% by mass or more and 10% by mass or less, more preferably 1% by mass or more and 8% by mass or less, further preferably 1% by mass or more and 5% by mass or less, and particularly preferably 2% by mass.

In the self-emulsifying composition of the present disclosure, a preferable combination of the surfactant, the auxiliary surfactant, and the cosolvent is not particularly limited as long as the sulfonamide derivative represented by the formula (1), a pharmaceutically acceptable salt thereof, or an active metabolite thereof can be dissolved and a self-emulsifying property is exhibited. However, for example, a combination of an ester of a fatty acid and a propylene glycol as the surfactant, an ester of a fatty acid and a macrogol glycerin or tocol derivative as the auxiliary surfactant, and ethanol as the cosolvent is preferable, and a combination of propylene glycol monolaurate or propylene glycol monocaprylate as the surfactant, polyoxyl 40 castor oil, polyoxyl 35 castor oil, or α-tocopherol PEG-1000 succinate as the auxiliary surfactant, and ethanol as the cosolvent is more preferable, and a combination of propylene glycol monocaprylate as the surfactant, polyoxyl 35 castor oil as the auxiliary surfactant, and ethanol as the cosolvent is particularly preferable.

In the self-emulsifying composition of the present disclosure, a preferable blending ratio of the surfactant, the auxiliary surfactant, and the cosolvent is not particularly limited as long as the sulfonamide derivative represented by the formula (1), a pharmaceutically acceptable salt thereof, or an active metabolite thereof can be dissolved and a self-emulsifying property is exhibited, but for example, the blending ratio in surfactant:auxiliary surfactant:cosolvent is preferably any of 45:45:10, 40:50:10, 30:60:10, 22.5:67.5:10, 10:80:10, 11:84:5, or 11:87:2, more preferably any of 10:80:10, 11:84:5, or 11:87:2, and particularly preferably 11:87:2.

The self-emulsifying composition of the present disclosure may further comprise an additive. The additive is not particularly limited as long as the additive is pharmaceutically acceptable and the self-emulsifying composition exhibits a self-emulsifying property, but the additive includes, for example, an excipient, a lubricant, a binder, a pH adjuster, a flavoring agent, a thickener, a buffering agent, a colorant, a diluent, a preservative, and/or a sweetener, and the like. As these additives, one of publicly-known additives may be used alone, or two or more of these may be used in combination. The blending amount of these additives may be in any desired ratio as appropriate as long as the self-emulsifying composition exhibits a self-emulsifying ability.

The self-emulsifying composition of the present disclosure can be pharmaceutically formulated by a publicly-known method as appropriate and used in treatment or prevention of diseases described later as a self-emulsifying drug delivery system. Preferably, as the step for pharmaceutical formulation, various pharmaceutical formulation techniques can be used as long as the self-emulsifying composition can be used as SEDDS, SMEDDS, and/or SNEDDS and exhibits a self-emulsifying property. More preferably, a pharmaceutical formulation technique that is capable of preparing a formulation suitable for oral administration can be selected. Here, the "formulation suitable for oral administration" refers to any of forms of a solution, a water-based or oil-based suspension, a syrup, an elixir, an emulsion, a tablet, a pill, pellets, granules, fine granules, a powder, a capsule containing granules, fine granules or a powder, a capsule containing a solution or a suspension, a sustained-release system, and the like. Further preferably, the formulation suitable for oral administration is a capsule, and may be either of a capsule containing fine granules or a powder or a capsule containing a solution or a suspension.

The capsule may be either of a hard capsule or a soft capsule but is preferably a soft capsule. The composition of the capsule film of the soft capsule is not necessarily limited, and the main component thereof includes, for example, gelatin, carrageenan, pectin, pullulan, sodium alginate, starch, hypromellose, hydroxypropyl cellulose, and the like, as well as various publicly-known components but is preferably gelatin. The gelatin is not particularly limited as long as the gelatin is commercially available.

As the self-emulsifying composition of the present disclosure contains the sulfonamide derivative represented by the formula (1), a pharmaceutically acceptable salt thereof, or an active metabolite thereof, the self-emulsifying composition of the present disclosure can be used as a treatment agent or prevention agent having an α4 integrin inhibiting action. Here, "treatment" refers to ameliorating clinical symptoms relating to diseases or disorders, that is, complete cure, reduction of the degree or frequency of clinical symptoms, or introduction to or maintenance of remission conditions, and includes actions conducted on living organisms of treatment subject toward these conditions, and the like, and "prevention" refers to reducing risks of developing diseases or disorders, and includes actions conducted on living organisms which have not contracted diseases or living organisms which are not exhibiting clinical symptoms to prevent the diseases from being developed, and the like.

Diseases which can be effectively treated or prevented by using the self-emulsifying composition of the present disclosure or a treatment agent or prevention agent containing the self-emulsifying composition include inflammatory diseases for which an α4 integrin-dependent adhesion process is involved in the pathologies. Specifically, such diseases are inflammatory diseases in which α4 integrin is involved in an adhesion process between blood vessel epithelial cells and inflammation-related cells, and more specifically, are diseases which can be treated or prevented by the sulfonamide derivative represented by the formula (1), a pharmaceutically acceptable salt thereof, or an active metabolite thereof, inhibiting α4 integrin to prevent inflammation-related cells from infiltrating from the blood vessel epithelium to tissues. Although not limited to these, such diseases include, for example, rheumatoid arthritis, ulcerative colitis, Crohn's disease, systemic lupus erythematosus, multiple sclerosis, Sjogren's syndrome, asthma, psoriasis, allergy, diabetes, cardiovascular disease, arteriosclerosis, restenosis, tumor growth, tumor metastasis, transplantation rejection, and/or human immunodeficiency virus infection, and the like.

When being orally administered in a treatment effective amount or an amount sufficient for treatments, the self-emulsifying drug delivery system or treatment agent of the present disclosure exhibits useful therapeutic effects on the above diseases. Here, the "treatment effective amount" and the "amount sufficient for treatments" have the same meaning, and refer to an amount effective or sufficient to exhibit the drug efficacy in the case where the pharmaceutical agent is administered to a living organism in order to treat a disease and can be determined variously depending on the gender, age, physical size, disease type, degree of the disease, lifestyle habit, and the like of a living organism.

The self-emulsifying composition, the self-emulsifying drug delivery system, the treatment agent, and/or the prevention agent of the present disclosure has properties of being emulsified when orally administered to a living organism and then mixed with the gastrointestinal tract fluid. In addition, emulsification allows the sulfonamide derivative represented by the formula (1) or a pharmaceutically acceptable salt thereof to be dissolved from an emulsion formed by the self-emulsifying drug delivery system. Here, the "gastrointestinal tract fluid" refers to a liquid component secreted from a living organism in the oral cavity, esophagus, stomach, small intestine, the large intestine, and the like or a liquid component present in the gastrointestinal tract, and specifically refers to saliva, gastric acid, bile, pancreatic juice, intestinal juice, and the like. Since these gastrointestinal tract fluids have various liquid properties from an acidic condition to a neutral condition, the self-emulsifying drug delivery system preferably has properties of allowing the compound to be dissolved from a formed emulsion while the self-emulsifying drug delivery system passes inside the gastrointestinal tract after oral administration, more preferably has properties of allowing the compound to be dissolved from a formed emulsion within a pH range from an acidic condition to a neutral condition exhibited by the gastrointestinal tract fluid, further preferably has properties of allowing the compound to be dissolved from a formed emulsion within a wide pH range from an acidic condition to a neutral condition, and particularly preferably has properties of allowing the compound to be dissolved from a formed emulsion within a wide pH range from an acidic condition to a neutral condition and allowing the compound to be dissolved more in an acidic condition than in a neutral condition. Here, the acidic condition is typically a condition mixed with the gastric acid in the stomach and generally refers to around pH 2.0 to 3.0. The neutral condition is typically a condition mixed with the bile, pancreatic juice, or intestinal juice in the small intestine or the large intestine and generally refers to around pH 6.0 to 7.5.

In one aspect of the present disclosure, for example, the self-emulsifying drug delivery system has properties of allowing the compound to be dissolved more in an acidic condition than the case where the sulfonamide derivative represented by the formula (1), a pharmaceutically acceptable salt thereof, or an active metabolite thereof is dissolved in an aqueous solution of methyl cellulose. In addition, in one aspect of the present disclosure, for example, the self-emulsifying drug delivery system has properties of allowing the compound to be dissolved more in an acidic condition than the case where the sulfonamide derivative represented by the formula (1), a pharmaceutically acceptable salt thereof, or an active metabolite thereof is dissolved in a polyethylene glycol solution.

The absorbability of the self-emulsifying composition, the self-emulsifying drug delivery system, the treatment agent, and/or the prevention agent in the gastrointestinal tract can be obtained directly or indirectly through an experimental evaluation. Specifically, the absorbability can be evaluated by exposing the self-emulsifying composition to the above acidic condition to neutral condition and can be evaluated in either of an in vitro experiment or an in vivo experiment. Although not limited to this, as the in vitro experiment, for example, a Tube method which will be described later can be used. In addition, although not limited to this, as the in vivo experiment, for example, a PO/PK experiment in which the blood is collected with time after the self-emulsifying composition is orally administered to rodents or non-rodents and then the blood concentration of an active pharmaceutical ingredient and/or an active metabolite thereof is analyzed can be used. Non-rodents are typically dogs, monkeys or humans. By plotting the blood concentration thus obtained on the vertical axis and the elapsed period of time on the horizontal axis, a curve indicating a change in blood concentration with time can be illustrated, and an area under the curve (AUC) of this curve can also be used for evaluation of the absorbability.

In one aspect of the present disclosure, for example, the self-emulsifying drug delivery system exhibits a higher AUC than the case where the sulfonamide derivative represented by the formula (1), a pharmaceutically acceptable salt thereof, or an active metabolite thereof is dissolved in an aqueous solution of methyl cellulose. In addition, in one aspect of the present disclosure, it is preferable that the self-emulsifying drug delivery system exhibit a higher AUC than the case where the sulfonamide derivative represented by the formula (1), a pharmaceutically acceptable salt thereof, or an active metabolite thereof is dissolved in a polyethylene glycol solution.

From the viewpoint of easily conducting the evaluation, it is preferable to use the Tube method as an in vitro experiment, for example. The Tube method is an experimental method as follows. A solution of a self-emulsifying composition in which a certain amount of a compound is dissolved is prepared, and is first added to a test liquid imitating an acidic condition in the stomach, and thereafter a small amount of the test liquid after a lapse of a desired period of time is sampled. Subsequently, the test liquid is added in a manner imitating the neutral condition of the small intestine, and the same operation is repeated. Subsequently, the test liquid is further added in a manner imitating the neutral condition of the large intestine, and the same operation is repeated. Then, an absorbability can be evaluated indirectly from a dissolution rate in each condition by quantifying the amount of the compound in each test liquid obtained as described above by a known analytical method such as a high-performance liquid chromatography method, a gas chromatography method, a supercritical fluid chromatography method, or a mass spectrometry method.

Here, the "dissolution rate" in the present Specification is such a numerical value that when the amount of a compound dissolved in a solution of a desired self-emulsifying composition is considered as 100%, after the solution is mixed with a certain test liquid, the numerical value is obtained by expressing the amount of the compound dissolved from a formed emulsion into the test liquid by percentage. In addition, by plotting the concentration of the compound in the test liquid obtained in each experiment condition on the vertical axis and the elapsed period of time on the horizontal axis, a curve indicating a change in the concentration of the compound in the test liquid with time can be illustrated, and an AUC of this curve can also be used for evaluation of the absorbability.

### Examples

Hereinafter, the invention of the present disclosure will be described in detail by using Examples.

### (Production Example 1) Preparation of Self-Emulsifying Compositions 1 to 84

Each surfactant, auxiliary surfactant, and cosolvent were weighed and mixed, and an active pharmaceutical ingredient (compound (1)) was added to a mixture thus obtained, followed by treatment using an ultrasonic homogenizer under 50°C to prepare self-emulsifying compositions 1 to 84 shown in Table 1 to Table 7. Each numerical value in Table 1 to Table 7 represents % by mass.

**Table 1**

| No. | 1 | 2 | 3 | 4 | 5 | 6 | 7 | 8 | 9 | 10 |
|---|---|---|---|---|---|---|---|---|---|---|
| Active pharmaceutical ingredient | 5 | 5 | 5 | 5 | 5 | 5 | 5 | 5 | 5 | 5 |
| Capryol 90 | 9.5 | 47.5 | 42.7 5 | 9.5 | 42.7 5 | 9.5 | - | 42.7 5 | 42.7 5 | 9.5 |
| Capmul MCM C8 | - | - | - | - | - | - | 42.7 5 | - | - | - |
| Labrasol ALF | 85.5 | 47.5 | 42.7 5 | 76 | - | - | - | 42.7 5 | - | - |
| Kolliphor EL | - | - | - | - | 42.7 5 | 76 | 42.7 5 | - | 42.7 5 | 76 |
| Propylene Glycol | - | - | 9.5 | 9.5 | 9.5 | 9.5 | 9.5 | - | - | - |
| Ethanol | - | - | - | - | - | - | - | 9.5 | 9.5 | 9.5 |

**Table 2**

| No. | 11 | 12 | 13 | 14 | 15 | 16 | 17 | 18 | 19 | 20 | 21 | 22 |
|---|---|---|---|---|---|---|---|---|---|---|---|---|
| Active pharmaceutical ingredient | 5 | 5 | 5 | 5 | 5 | 5 | 5 | 5 | 5 | 5 | 5 | 5 |
| Capryol 90 | 42. 75 | 38 | 28. 5 | 21. 375 | 9.5 | 47. 5 | 47. 5 | - | - | 57 | 66. 5 | 76 |
| Capmul MCM C8 | - | - | - | - | - | - | - | 47. 5 | 47. 5 | - | - | - |
| Kolliphor EL | - | 38 | 28. 5 | 21. 375 | 9.5 | 47. 5 | | 47. 5 | | 28. 5 | 19 | 9.5 |
| Tween 80 | - | - | - | - | - | - | 47. 5 | - | 47. 5 | - | - | - |
| TPGS | 42. 75 | 9.5 | 28. 5 | 42. 75 | 66.5 | - | - | - | - | - | - | - |
| Ethanol | 9.5 | 9.5 | 9.5 | 9.5 | 9.5 | - | - | - | - | 9.5 | 9.5 | 9.5 |

**Table 3**

| No. | 23 | 24 | 25 | 26 | 27 | 28 | 29 | 30 | 31 | 32 | 33 | 34 |
|---|---|---|---|---|---|---|---|---|---|---|---|---|
| Active pharmaceutical ingredient | 10 | 10 | 10 | 10 | 10 | 10 | 10 | 10 | 10 | 10 | 10 | 10 |
| Capryol 90 | 40. 5 | 36 | 27 | 20. 25 | 9 | 40. 5 | 9 | 40. 5 | 40. 5 | - | - | 40. 5 |
| Capmul MCM C8 | - | - | - | - | - | - | - | - | - | 40. 5 | 45 | - |
| Kolliphor EL | - | 36 | 27 | 20. 25 | 9 | 40. 5 | 72 | 40. 5 | 40. 5 | - | - | - |
| Kolliphor RH40 | - | - | - | - | - | - | - | - | - | 40. 5 | 45 | 40. 5 |
| TPGS | 40. 5 | 9 | 27 | 40. 5 | 63 | - | - | - | - | - | - | - |
| Propylene Glycol | - | - | - | - | - | - | - | - | 9 | - | - | - |
| Propylene Carbonate | - | - | - | - | - | - | - | 9 | - | - | - | - |
| Ethanol | 9 | 9 | 9 | 9 | 9 | 9 | 9 | - | - | 9 | - | 9 |

**Table 4**

| No. | 35 | 36 | 37 | 38 | 39 | 40 | 41 | 42 | 43 | 44 | 45 | 46 |
|---|---|---|---|---|---|---|---|---|---|---|---|---|
| Active pharmaceutical ingredient | 10 | 10 | 10 | 10 | 10 | 10 | 10 | 10 | 10 | 10 | 10 | 10 |
| Capryol 90 | | 40. | 40. | 40. | - | - | - | - | - | - | - | - |
| | | 5 | 5 | 5 | | | | | | | | |
| Capmul MCM C8 | 40. 5 | - | - | - | - | - | - | - | - | - | - | - |
| Plurol Oleique CC497 | | | | | 40. 5 | 40. 5 | 40. 5 | 40. 5 | 40. 5 | | | |
| Lauroglycol 90 | - | - | - | - | - | - | - | - | - | 40. 5 | 40. 5 | 40. 5 |
| Labrasol ALF | - | - | - | - | - | 40. 5 | - | - | - | - | - | - |
| Kolliphor EL | 40. 5 | - | - | - | - | - | - | - | - | - | 40. 5 | - |
| Kolliphor RH40 | - | - | - | - | - | - | - | - | - | - | - | 40. 5 |
| Kolliphor HS15 | - | - | - | - | - | - | - | - | - | 40. 5 | - | - |
| Gelucire 44/14 | - | 40. 5 | - | - | - | - | 40. 5 | - | - | - | - | - |
| Gelucire 48/16 | - | - | 40. 5 | - | - | - | - | - | - | - | - | - |
| Gelucire 50/13 | - | - | - | 40. 5 | - | - | - | - | - | - | - | - |
| TPGS | - | - | - | - | - | - | - | 40. 5 | - | - | - | - |
| Tween 80 | - | - | - | - | 40. 5 | - | - | - | - | - | - | - |
| BRIJ S10 | - | - | - | - | - | - | - | - | 40. 5 | - | - | - |
| Ethanol | 9 | 9 | 9 | 9 | 9 | 9 | 9 | 9 | 9 | 9 | 9 | 9 |

**Table 5**

| No. | 47 | 48 | 49 | 50 | 51 | 52 | 53 | 54 | 55 | 56 | 57 | 58 |
|---|---|---|---|---|---|---|---|---|---|---|---|---|
| Active pharmaceutical ingredient | 10 | 10 | 10 | 10 | 10 | 10 | 10 | 10 | 10 | 10 | 10 | 10 |
| Lauroglycol 90 | 40. 5 | 40. 5 | 40. 5 | 40. 5 | 40. 5 | 40. 5 | - | - | - | - | - | - |
| Lauroglycol FCC | - | - | - | - | - | - | 40. 5 | 40. 5 | 40. 5 | 40. 5 | 40. 5 | 40. 5 |
| Labrasol ALF | - | 40. 5 | - | - | - | - | - | - | - | - | 40. 5 | - |
| Kolliphor EL | - | - | - | - | - | - | - | 40. 5 | - | - | - | - |
| Kolliphor RH40 | - | - | - | - | - | - | - | - | 40. 5 | - | - | - |
| Kolliphor HS15 | - | - | - | - | - | - | 40. 5 | - | - | - | - | - |
| Gelucire 44/14 | - | - | 40. 5 | - | - | - | - | - | - | - | - | 40. 5 |
| Gelucire 50/13 | - | - | - | 40. 5 | - | - | - | - | - | - | - | - |
| TPGS | - | - | - | - | 40. 5 | - | - | - | - | - | - | - |
| Tween 80 | 40. 5 | - | - | - | - | - | - | - | - | 40. 5 | - | - |
| BRIJ S10 | - | - | - | - | - | 40. 5 | - | - | - | - | - | - |
| Ethanol | 9 | 9 | 9 | 9 | 9 | 9 | 9 | 9 | 9 | 9 | 9 | 9 |

**Table 6**

| No. | 59 | 60 | 61 | 62 | 63 | 64 | 65 | 66 | 67 | 68 | 69 | 70 |
|---|---|---|---|---|---|---|---|---|---|---|---|---|
| Active pharmaceutical ingredient | 10 | 10 | 10 | 10 | 10 | 10 | 10 | 10 | 10 | 10 | 10 | 10 |
| Lauroglycol FCC | 40. 5 | 40. 5 | 40. 5 | - | - | - | - | - | - | - | - | - |
| Capryol PGMC | - | - | - | 40. 5 | 40. 5 | 40. 5 | 40. 5 | 40. 5 | 40. 5 | 40. 5 | 40. 5 | 40. 5 |
| Labrasol ALF | - | - | - | - | - | - | - | 40. 5 | - | - | - | - |
| Kolliphor EL | - | - | - | - | 40. 5 | - | - | - | - | - | - | - |
| Kolliphor RH40 | - | - | - | - | - | 40. 5 | - | - | - | - | - | - |
| Kolliphor HS15 | - | - | - | 40. 5 | - | - | - | - | - | - | - | - |
| Gelucire 44/14 | - | - | - | - | - | - | - | - | 40. 5 | - | - | - |
| Gelucire 50/13 | 40. 5 | - | - | - | - | - | - | - | - | 40. 5 | - | - |
| TPGS | - | 40. 5 | - | - | - | - | - | - | - | - | 40. 5 | - |
| Tween 80 | - | - | - | - | - | - | 40. 5 | - | - | - | - | - |
| BRIJ S10 | - | - | 40. 5 | - | - | - | - | - | - | - | - | 40. 5 |
| Ethanol | 9 | 9 | 9 | 9 | 9 | 9 | 9 | 9 | 9 | 9 | 9 | 9 |

**Table 7**

| No. | 71 | 72 | 73 | 74 | 75 | 76 | 77 | 78 | 79 | 80 | 81 | 82 | 83 | 84 |
|---|---|---|---|---|---|---|---|---|---|---|---|---|---|---|
| Active pharmaceutical ingredient | 10 | 10 | 10 | 10 | 10 | 10 | 10 | 10 | 10 | 10 | 10 | 10 | 10 | 10 |
| Capryol 90 | 40. 5 | 40. 5 | 40. 5 | 40. 5 | 40. 5 | 40. 5 | - | 40. 5 | - | - | - | - | - | - |
| Capmul MCM C8 | - | - | - | - | - | - | 40. 5 | - | 40. 5 | 40. 5 | 40. 5 | 40. 5 | 40. 5 | 40. 5 |
| Labrasol ALF | - | - | - | - | - | - | - | 40. 5 | - | 40. 5 | - | - | - | - |
| Kolliphor HS15 | 40. 5 | - | - | - | - | - | 40. 5 | - | - | - | - | - | - | - |
| Gelucire 44/14 | - | - | - | - | - | - | - | - | - | - | 40. 5 | - | - | - |
| Gelucire 50/13 | - | - | - | - | - | - | - | - | - | - | - | 40. 5 | - | - |
| TPGS | - | - | - | - | - | - | - | - | - | - | - | - | 40. 5 | - |
| Tween 80 | - | - | - | 40. 5 | - | - | - | - | 40. 5 | - | - | - | - | - |
| BRIJ S10 | - | - | - | - | 40. 5 | - | - | - | - | - | - | - | - | 40. 5 |
| LABRAFIL M2125CS | - | 40. 5 | - | - | - | - | - | - | - | - | - | - | - | - |
| LABRAFIL M1944CS | - | - | 40. 5 | - | - | - | - | - | - | - | - | - | - | - |
| Sucrose myristate | - | - | - | - | - | 40. 5 | - | - | - | - | - | - | - | - |
| Ethanol | 9 | 9 | 9 | 9 | 9 | 9 | 9 | 9 | 9 | 9 | 9 | 9 | 9 | 9 |

The surfactants stated in the above Tables 1 to 7 are as follows.
- Capryol 90: Propylene glycol monocaprylate (type II)
- Capmul MCM C8: Glyceryl monocaprylate
- Plurol Oleique CC497: Polyglyceryl-3-dioleate
- Lauroglycol 90: Propylene glycol monolaurate (type II)
- Lauroglycol FCC: Propylene glycol monolaurate (type I)
- Capryol PGMC: Propylene glycol monocaprylate (type I)

The auxiliary surfactants stated in the above Tables 1 to 7 are as follows.
- Labrasol ALF: PEG-8 caprylic/capric glycerides
- Kolliphor EL: Polyoxyl 35 castor oil
- Kolliphor RH40: Polyoxyl 40 castor oil
- Kolliphor HS15: Polyglyceryl-15 hydroxystearate
- Gelucire 44/14: Lauroyl polyoxyl-32 glycerides
- Gelucire 48/16: Stearoyl polyoxyl-32 glycerides
- Gelucire 50/13: Stearoyl polyoxyl-32 glycerides
- TPGS: d-α-Tocopherol PEG-1000 succinate
- Tween 80: Polysorbate 80
- BRIJ S10: Polyethylene glycol octadecyl ether
- LABRAFIL M2125CS: Linoleoyl polyoxyl-6 glycerides
- LABRAFIL M1944CS: Oleoyl polyoxyl-6 glycerides
- Sucrose myristate

Among the self-emulsifying compositions in Production Example 1, correspondences of self-emulsifying compositions that have the same component composition except for the concentrations, and have the same component ratio except for the active pharmaceutical ingredients are shown in Table 8. The self-emulsifying compositions 85 to 87 were prepared in conformity with Production Example 1. The self-emulsifying composition 85 contained 1.5% by mass of the active pharmaceutical ingredient, 44.325% by mass of Capryol 90, 44.325% by mass of Labrasol ALF, and 9.85% by mass of ethanol. The self-emulsifying composition 86 contained 1.5% by mass of the active pharmaceutical ingredient, 44.325% by mass of Capryol 90, 44.325% by mass of Kolliphor EL, and 9.85% by mass of ethanol. The self-emulsifying composition 87 contained 1.5% by mass of the active pharmaceutical ingredient, 9.85% by mass of Capryol 90, 78.8% by mass of Kolliphor EL, and 9.85% by mass of ethanol.

**Table 8**

| | Active pharmaceutical ingredient concentration | | | Ratio of components except for active pharmaceutical ingredient (%) | | | | |
|---|---|---|---|---|---|---|---|---|
| | 15 mg/g | 50 mg/g | 100 mg/g | Capryol 90 | Labrasol ALF | Kolliphor EL | TP GS | Ethan ol |
| | 85 | 8 | 78 | 45 | 45 | | | 10 |
| | 86 | 9 | 28 | 45 | | 45 | | 10 |
| Self-emuls ifying compositi on No. | 87 | 10 | 29 | 10 | | 80 | | 10 |
| | | 11 | 23 | 45 | | | 45 | 10 |
| | | 12 | 24 | 40 | | 40 | 10 | 10 |
| | | 13 | 25 | 30 | | 30 | 30 | 10 |
| | | 14 | 26 | 22.5 | | 22.5 | 45 | 10 |
| | | 15 | 27 | 10 | | 10 | 70 | 10 |

### (Production Example 2) Preparation of Comparative Example Compositions

Polyethylene glycol 400 was weighed in an amount of 1 g, and the active pharmaceutical ingredient was added thereto in a specified concentration, followed by treatment using an ultrasonic homogenizer under 50°C to prepare PEG solutions having active pharmaceutical ingredient concentrations of 15 mg/g (PEG solution 1), 50 mg/g (PEG solution 2), and 100 mg/g (PEG solution 3). In addition, to 1 g of 0.5% aqueous solution of methyl cellulose prepared by adding and dissolving 125 mg of methyl cellulose 400 to 25 mL of purified water, the active pharmaceutical ingredient was added in a specified concentration, followed by treatment using an ultrasonic homogenizer under 50°C to prepare MC solutions having an active pharmaceutical ingredient concentration of 15 mg/g (MC solution 1), an active pharmaceutical ingredient concentration of 50 mg/g (MC solution 2), and an active pharmaceutical ingredient concentration of 100 mg/g (MC solution 3).

### (Evaluation 1-1) Dissolution test: Tube method

To 1.5 mL of a HCl/saline aqueous solution adjusted to pH 2.0, 1.0 mL of a 0.5% aqueous solution of methyl cellulose was added, followed by shaking at 37°C and 90 rpm to obtain a test liquid. To this test liquid, 100 mg of the self-emulsifying composition 1 to 84 prepared in Production Example 1, the PEG solution 2 or 3 prepared in Production Example 2, or the MC solution 2 was added as a test sample and mixed by inversion, followed by shaking at 37°C and 90 rpm. Then, 500 µL of the test liquid was sampled 15 minutes after the test sample was added, and the solution thus sampled was promptly filtered by using a filtration vial integrated with filter (Mini-UniPrep), pore size. 45 µm) and centrifuged to obtain a supernatant. To 100 µL of the supernatant, 100 µL of acetonitrile and 600 µL of a water/acetonitrile mixture liquid (1:1) were added to obtain a stomach stage sample (pH 2.4). After the stomach stage sample was sampled, 7.5 mL of FaSSIF, which had been heated to 37°C in advance, was added, followed by shaking at 37°C and 90 rpm. Then, 500 µL of the test liquid was sampled 30 minutes after or 30 minutes after, 60 minutes after, 90 minutes after, and 120 minutes after FaSSIF was added, the solution thus sampled was promptly filtered by using the filtration vial integrated with filter (Mini-UniPrep), pore size. 45 µm) and centrifuged to obtain a supernatant. To 200 µL of the supernatant, 200 µL of acetonitrile was added to obtain a small intestine stage sample (pH 6.4). Each sample was analyzed through high-performance liquid chromatography to measure the dissolution rate of the drug at the stomach or small intestine stage. The analysis conditions for the high-performance liquid chromatography were as follows.

### <HPLC>

Detector: Ultraviolet absorptiometer (measurement wavelength :264 nm)
Column: Stainless steel pipe having an inner diameter of 3.0 mm and a length of 150 mm filled with an octadecylsilyl silica gel for liquid chromatography having a particle size of 2.7 µm.
Column temperature: Constant temperature around 30°C
Flow rate: 0.5 mL/min
Area measurement range: 6 minutes after the sample was injected
Mobile phase: 25mM Ammonium acetate solution (pH 5.0)/acetonitrile mixture liquid (13:12)

The dissolution rates at pH 2.4 or pH 6.4 regarding the respective self-emulsifying compositions 1 to 84 are shown in Tables 9 to 15. In addition, numerical values (Tube method AUC_{0-2.25}) of AUC of curves obtained by plotting the time (0 to 2.25 hours) on the horizontal axis and the dissolution rates on the vertical axis and logarithmic values (Tube method Log AUC_{0-2.25}) thereof regarding the self-emulsifying compositions 1 to 38 are shown in Tables 9 to 12. Moreover, dissolution rates at pH 2.4 or pH 6.4 as well as numerical values (Tube method AUC_{0-2.25}) of AUC of curves obtained by plotting the time (0 to 2.25 hours) on the horizontal axis and the dissolution rates on the vertical axis and logarithmic value (Tube method Log AUC_{0-2.25}) thereof regarding the respective PEG solution 2, PEG solution 3, and MC solution 2 are shown in Table 16.

**Table 9**

| No. | 1 | 2 | 3 | 4 | 5 | 6 | 7 | 8 | 9 | 10 |
|---|---|---|---|---|---|---|---|---|---|---|
| Dissolution rate (pH 2.4) | 10.6 | 42.0 | 62.1 | 6.4 | 82.7 | 60.1 | 0.6 | 2.8 | 84.0 | 59.7 |
| Dissolution rate (pH 6.4) | 43.0 | 7.5 | 6.8 | 36.7 | 81.1 | 87.6 | 72.2 | 8.0 | 85.4 | 81.6 |
| Tube method AUC_{0-2.25} | 406. 4 | 367. 2 | 518. 0 | 323. 3 | 1219 .9 | 1100 .8 | 534. 1 | 79.9 | 1258 .9 | 1048 .3 |
| Tube method Log(AUC_{0-2.25}) | 6.0 | 5.9 | 6.3 | 5.8 | 7.1 | 7.0 | 6.3 | 4.4 | 7.1 | 7.0 |

**Table 10**

| No. | 11 | 12 | 13 | 14 | 15 | 16 | 17 | 18 | 19 | 20 | 21 | 22 |
|---|---|---|---|---|---|---|---|---|---|---|---|---|
| Dissolution rate (pH 2.4) | 42. 0 | 97. 9 | 85. 0 | 80. 0 | 57. 1 | 20. 9 | 20. 9 | 1.1 | 29. 1 | 50. 3 | 19. 3 | 6.8 |
| Dissolution rate (pH 6.4) | 68. 9 | 90. 0 | 80. 5 | 91. 9 | 69. 9 | 34. 2 | 56. 9 | 78. 1 | 79. 6 | 101 .6 | 96. 2 | 30. 9 |
| Tube method AUC_{0-2.25} | 771 .1 | 139 2.0 | 122 2.1 | 126 0.9 | 938 .3 | 430 .3 | 576 .7 | 573 .7 | 810 .9 | 112 6.8 | 745 .1 | 250 .3 |
| Tube method Log(AUC_{0-2.25}) | 6.6 | 7.2 | 7.1 | 7.1 | 6.8 | 6.1 | 6.4 | 6.4 | 6.7 | 7.0 | 6.6 | 5.5 |

**Table 11**

| No. | 23 | 24 | 25 | 26 | 27 | 28 | 29 | 30 | 31 | 32 | 33 | 34 |
|---|---|---|---|---|---|---|---|---|---|---|---|---|
| Dissolution rate (pH 2.4) | 17. 0 | 49. 8 | 32. 0 | 28. 2 | 27. 2 | 56. 8 | 31. 5 | 59. 8 | 62. 8 | 9.2 | 12. 1 | 35. 2 |
| Dissolution rate (pH 6.4) | 30. 2 | 43. 8 | 42. 1 | 48. 4 | 57. 5 | 49. 9 | 58. 4 | 44. 7 | 46. 8 | 34. 1 | 38. 6 | 33. 6 |
| Tube method AUC_{0-2.25} | 705 .5 | 138 7.5 | 110 0.8 | 113 7.2 | 125 9.1 | 158 3.4 | 132 8.2 | 154 0.8 | 162 2.3 | 642 .9 | 757 .2 | 101 6.1 |
| Tube method Log(AUC_{0-2.25}) | 6.6 | 7.2 | 7.0 | 7.0 | 7.1 | 7.4 | 7.2 | 7.3 | 7.4 | 6.5 | 6.6 | 6.9 |

**Table 12**

| No. | 35 | 36 | 37 | 38 | 39 | 40 | 41 | 42 | 43 | 44 | 45 | 46 |
|---|---|---|---|---|---|---|---|---|---|---|---|---|
| Dissolution rate (pH 2.4) | 0.5 | 11. 4 | 9.4 | 13. 4 | 1.0 | 0.8 | 1.1 | 0.9 | 2.1 | 2.1 | 41. 3 | 45. 0 |
| Dissolution rate (pH 6.4) | 41. 1 | 21. 4 | 19. 4 | 25. 4 | 16. 2 | 6.3 | 15. 1 | 6.8 | 17. 9 | 21. 3 | 52. 9 | 53. 6 |
| Tube method AUC_{0-2.25} | 583 .5 | 486 .4 | 424 .2 | 575 .5 | | | | | | | | |
| Tube method Log(AUC_{0-2.25}) | 6.4 | 6.2 | 6.1 | 6.4 | | | | | | | | |

**Table 13**

| No. | 47 | 48 | 49 | 50 | 51 | 52 | 53 | 54 | 55 | 56 | 57 | 58 |
|---|---|---|---|---|---|---|---|---|---|---|---|---|
| Dissolution rate (pH 2.4) | 4.1 | 13. 1 | 15. 3 | 2.9 | 8.3 | 5.1 | 15. 6 | 29. 1 | 28. 9 | 27. 6 | 1.3 | 18. 5 |
| Dissolution rate (pH 6.4) | 30. 7 | 18. 1 | 32. 7 | 21. 6 | 44. 6 | 41. 8 | 44. 6 | 49. 6 | 46. 4 | 48. 3 | 22. 0 | 33. 9 |

**Table 14**

| No. | 59 | 60 | 61 | 62 | 63 | 64 | 65 | 66 | 67 | 68 | 69 | 70 |
|---|---|---|---|---|---|---|---|---|---|---|---|---|
| Dissolution rate (pH 2.4) | 6.8 | 21. 7 | 7.8 | 28. 8 | 42. 8 | 32. 6 | 22. 0 | 3.0 | 15. 4 | 20. 8 | 26. 9 | 19. 1 |
| Dissolution rate (pH 6.4) | 37. 2 | 59. 2 | 43. 6 | 41. 9 | 54. 2 | 46. 2 | 44. 6 | 22. 5 | 32. 0 | 36. 8 | 47. 4 | 43. 6 |

**Table 15**

| No. | 71 | 72 | 73 | 74 | 75 | 76 | 77 | 78 | 79 | 80 | 81 | 82 | 83 | 84 |
|---|---|---|---|---|---|---|---|---|---|---|---|---|---|---|
| Dissolution rate (pH 2.4) | 13. 5 | 1.2 | 1.1 | 12. 5 | 10. 0 | 1.1 | 1.1 | 1.0 | 7.5 | 1.1 | 1.7 | 4.3 | 25. 3 | 5.8 |
| Dissolution rate (pH 6.4) | 40. 0 | 4.8 | 4.8 | 29. 1 | 35. 1 | 11. 7 | 23. 9 | 8.8 | 33. 7 | 11. 1 | 27. 4 | 26. 2 | 42. 7 | 25. 8 |

**Table 16**

| No. | PEG solution 2 | PEG solution 3 | MC solution 2 |
|---|---|---|---|
| Dissolution rate (pH 2.4) | 1.0 | 0.6 | 0.7 |
| Dissolution rate (pH 6.4) | 21.8 | 12.7 | 20.5 |
| Tube method AUC_{0-2.25} | 165.8 | 199.7 | 156.4 |
| Tube method Log(AUC_{0-2.25}) | 5.1 | 5.3 | 5.1 |

### (Evaluation 1-2) Dissolution test: Tube method

For the self-emulsifying compositions 85 to 87 and a self-emulsifying composition (hereinafter referred to as a self-emulsifying composition 88) prepared in the same manner as in the self-emulsifying composition 30 except that 10% by mass of the active pharmaceutical ingredient, 11% by mass of Capryol 90, 77.2% by mass of Kolliphor EL, and 1.8% by mass of ethanol were contained, as well as the MC solution 1 and the MC solution 3 prepared in Production Example 2, the above dissolution test was conducted to measure dissolution rates, and numerical values (Tube method AUC_{0-2.25}) of AUC and logarithmic values (Tube method Log AUC_{0-2.25}) thereof were calculated. Results are shown in Table 17.

**Table 17**

| No. | MC solution 1 | MC solution 3 | 85 | 86 | 87 | 88 |
|---|---|---|---|---|---|---|
| Dissolution rate (pH 2.4) | 0.9 | 0.1 | 17.7 | 69.4 | 3.8 | 31.4 |
| Dissolution rate (pH 6.4) | 40.0 | 8.4 | 15.4 | 92.2 | 68.3 | 53.3 |
| Tube method AUC_{0-2.25} | 85.7 | 125.9 | 80.7 | 356.8 | 161.3 | 1245.0 |
| Tube method Log(AUC_{0-2.25}) | 4.5 | 4.8 | 4.4 | 5.9 | 5.1 | 7.1 |

### (Evaluation 2-1) Blood plasma concentration evaluation 1 through PO/PK experiment using rats

Each of the self-emulsifying compositions 85 to 87 was orally administered to each group of 3 male rats (Crl:CD(SD)) under fasting conditions, and transition of the blood plasma concentration of the active metabolite of the active pharmaceutical ingredient was evaluated. Note that the fasting conditions were such that fasting was started about 15 hours or more before the administration. To each animal, the self-emulsifying composition in an amount equivalent to 3 mg/kg of the active pharmaceutical ingredient was administered. The blood was collected from the subclavian vein 0.25 hours, 0.5 hours, 1 hour, 2 hours, 4 hours, 6 hours, 8 hours, 10 hours, and 24 hours after the administration, and the blood plasma was sampled through a centrifugation operation. After a deproteinizing treatment was conducted on the blood plasma by using acetonitrile, the concentration of the active metabolite in the blood plasma was measured by using LC-MS/MS (API4000, manufactured by AB SCIEX). In addition, as control group, test animals to which the PEG solution 1 or the MC solution 1 prepared in Production Example 2 was administered were used. Tmax, Cmax, and AUC of the concentration of the active metabolite in the blood plasma thus obtained are shown in Table 18.

**Table 18**

| No. | Tmax (hr) | Cmax (ng/mL) | AUC (hr*ng/mL) |
|---|---|---|---|
| PEG solution 1 | 2.00 | 209 | 896 |
| MC solution 1 | 0.25 | 129 | 643 |
| 85 | 4.67 | 91.5 | 679 |
| 86 | 4.67 | 276 | 1645 |
| 87 | 3.33 | 242 | 1515 |

### (Evaluation 2-2) Blood plasma concentration evaluation 2 through PO/PK experiment using rats

Transition of the total concentration of the active pharmaceutical ingredient and the active metabolite thereof in the blood plasma was evaluated in the same manner as in Evaluation 2-1 except that the self-emulsifying composition 28 or 29 in an amount equivalent to 100 mg/kg or 300 mg/kg of the active pharmaceutical ingredient was administered to each animal. In addition, as control group, test animals to which the PEG solution 3 prepared in Production Example 2 was administered were used. Cmax and AUC of the concentration of the active pharmaceutical ingredient and the active metabolite in the blood plasma obtained in the case where the dosage was 100 mg/kg are shown in Table 19. In addition, Cmax and AUC of the concentration of the active pharmaceutical ingredient and the active metabolite in the blood plasma obtained in the case where the dosage was 300 mg/kg are shown in Table 20.

**Table 19**

| No. | Cmax (ng/mL) | AUC (hr*ng/mL) |
|---|---|---|
| PEG solution 3 | 1731±557 | 12052±2958 |
| 28 | 5840±2131 | 30342±8004 |
| 29 | 19224±5297 | 82017±26354 |

**Table 20**

| No. | Cmax (ng/mL) | AUC (hr*ng/mL) |
|---|---|---|
| PEG solution 3 | 2943±1301 | 24041±9053 |
| 28 | 8725±1880 | 44461±5071 |
| 29 | 30875±13990 | 122348±43087 |

### (Evaluation 3) Blood plasma concentration evaluation through PO/PK experiment using dogs

Each of the self-emulsifying composition 28 and the self-emulsifying composition 88 prepared in Production Example 1 was orally administered to 3 male beagles (10 to 15 months old) under fasting conditions, and transition of the blood plasma concentration of the active pharmaceutical ingredient and the active metabolite was evaluated. Note that the fasting conditions were such that fasting was started 16 hours or more before the administration. To each animal, a gelatin capsule was filled with the self-emulsifying composition in an amount equivalent to 100 mg/kg of the active pharmaceutical ingredient and administered. The blood was collected from the cephalic vein 0.25 hours, 0.5 hours, 1 hour, 2 hours, 4 hours, 6 hours, 8 hours, 10 hours, and 24 hours after the administration, and the blood plasma was sampled and treated. The total concentration of the active pharmaceutical ingredient and the active metabolite in the blood plasma was measured by using LC-MS/MS (API4000, manufactured by AB SCIEX). In addition, as control group, test animals to which the PEG solution 3 prepared in Production Example 2 was administered were used. Tmax, Cmax, and AUC of the concentration of the active pharmaceutical ingredient and the active metabolite in the blood plasma thus obtained are shown in Table 21.

**Table 21**

| No. | Tmax (hr) | Cmax (nmol/L) | AUC (hr*nmol/L) |
|---|---|---|---|
| PEG solution 3 | 1 | 1924 | 8593 |
| 28 | 0.67 | 5050 | 21852 |
| 88 | 1.67 | 6671 | 26274 |

### (Evaluation 4) Correlation of Tube method and PO/PK experiment

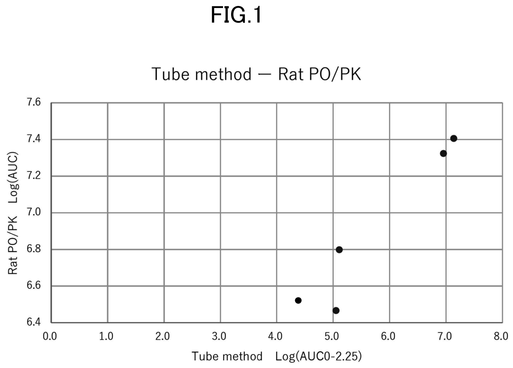

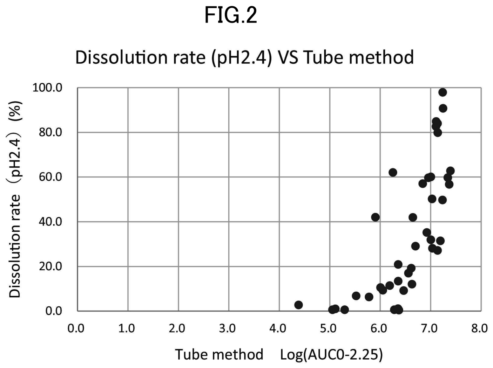

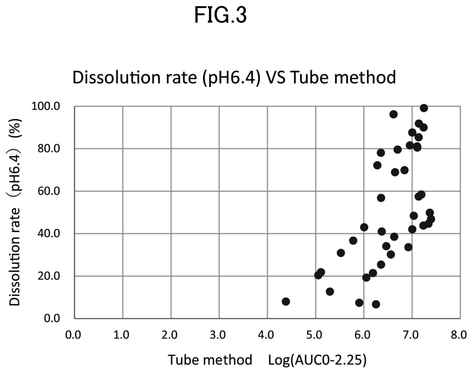

Results obtained by plotting the logarithmic values (Tube method Log AUC_{0-2.25}) of the AUC in the Tube method obtained in Evaluation 1 on the horizontal axis and the logarithmic values (rat PO/PK Log AUC) of the AUC of the blood plasma concentration obtained in Evaluation 2 on the vertical axis are shown in Fig. 1. In addition, results obtained by plotting the logarithmic values (Tube method Log AUC_{0-2.25}) of AUC in the Tube method obtained in Evaluation 1 on the horizontal axis and the dissolution rates (%) at pH 2.4 or pH 6.4 on the vertical axis are shown in Fig. 2 or Fig. 3. In addition, the respective correlation coefficients in Fig. 1 to Fig. 3 are shown in Table 22. AUC_{0-2.25} of the Tube method and AUC of rat PO/PK have a strong positive correlation, and AUC_{0-2.25} of the Tube method and the dissolution rates at pH 2.4 and pH 6.4 have a positive correlation. It is possible to surmise the pharmacokinetics (AUC) in the in vivo evaluation from the results of AUC_{0-2.25} of the Tube method or the dissolution rate at pH 2.4 or pH 6.4.

**Table 22**

| | Correlation coefficient |
|---|---|
| Tube method Log (AUC_{0-2.25}) vs rat PO/PK Log (AUC) | 0.961 |
| Dissolution rate (pH 2.4) vs Tube method Log (AUC_{0-2.25}) | 0.708 |
| Dissolution rate (pH 6.4) vs Tube method Log (AUC_{0-2.25}) | 0.640 |

The self-emulsifying composition of the present disclosure can ameliorate absorbability of the sulfonamide derivative represented by the formula (1) after oral administration.

## Claims

1. A self-emulsifying composition comprising: a sulfonamide derivative represented by the following formula (1), a pharmaceutically acceptable salt thereof, or an active metabolite thereof; and at least two solubilizers

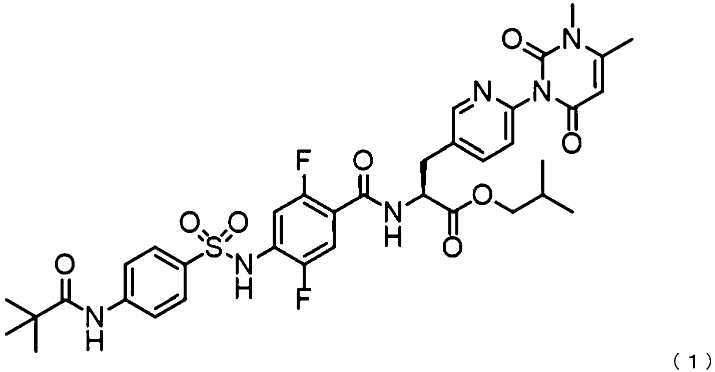

2. The self-emulsifying composition according to claim 1, wherein the at least two solubilizers comprise at least one surfactant and at least one auxiliary surfactant.

3. The self-emulsifying composition according to claim 1, wherein the solubilizers comprise at least two selected from a cyclodextrin or a derivative thereof, bile acid or a derivative thereof, a fatty alcohol, a fatty acid or derivatives thereof, and a tocol derivative.

4. The self-emulsifying composition according to claim 2, wherein the surfactant comprises at least one selected from the group consisting of polyglyceryl-2 dioleate, polyglyceryl-3 dioleate, polyglyceryl-10 trioleate, propylene glycol monolaurate, propylene glycol monocaprylate, glyceryl monocaprylate, glyceryl monolaurate, glyceryl dilaurate, and glyceryl monolinoleate.

5. The self-emulsifying composition according to claim 2, wherein the auxiliary surfactant comprises at least one selected from the group consisting of polyoxyl 40 castor oil, polyoxyl 35 castor oil, PEG-25 trioleate, PEG-60 corn glycerides, PEG-60 almond oil, PEG-40 palm kernel oil, PEG-50 castor oil, PEG-50 hydrogenated castor oil, PEG-60 hydrogenated castor oil, PEG-8 caprylic/capric glycerides, lauroyl polyoxyl-32 glycerides, linoleoyl polyoxyl-6 glycerides, oleoyl polyoxyl-6 glycerides, stearoyl polyoxyl-32 glycerides, PEG-6 caprylic/capric glycerides, polyglyceryl-10 monooleate, polyglyceryl-10 laurate, polyglyceryl-15 hydroxystearate, α-tocopherol PEG-400 succinate, α-tocopherol PEG-1000 succinate, dl-α-tocopherol PEG-1000 succinate, and d-α-tocopherol PEG-1000 succinate.

6. The self-emulsifying composition according to any one of claims 2, 4, and 5, wherein
a content of the surfactant is 0.1 times or more relative to % by mass of the sulfonamide derivative represented by the formula (1) or pharmaceutically acceptable salt thereof, and
a content of the auxiliary surfactant is 10 times or less relative to the sulfonamide derivative represented by the formula (1) or pharmaceutically acceptable salt thereof.

7. The self-emulsifying composition according to any one of claims 2, 4, and 5, wherein a ratio of the surfactant to the auxiliary surfactant is 1:1 to 8:1.

8. The self-emulsifying composition according to claim 2 or 4, wherein the surfactant comprises propylene glycol monocaprylate.

9. The self-emulsifying composition according to claim 2 or 5, wherein the auxiliary surfactant comprises polyoxyl 40 castor oil, polyoxyl 35 castor oil, or polyoxyethylene castor oil.

10. The self-emulsifying composition according to claim 1 or 2, further comprising a cosolvent.

11. A self-emulsifying drug delivery system in a form of a formulation suitable for oral administration, comprising the self-emulsifying composition according to claim 1 or 2.

12. The self-emulsifying drug delivery system according to claim 11, wherein the formulation is a capsule.
